# EUROPÄISCHE PATENTSCHRIFT

(11) **EP 1 683 473 B1**
(45) Veröffentlichungstag und Bekanntmachung des Hinweises auf die Patenterteilung: **28.11.2007**
(21) Anmeldenummer: 06000772.1
(22) Anmeldetag: 14.01.2006
(51) Int. Cl.: A61B 1/31

(54) **Einrichtung zur Verwendung bei der Behandlung eines Hämorrhoidenprolaps**
Device for use in treatment of hemorrhoidal prolapse
Dispositif destiné à être utilisé pour traiter un prolapsus hémorroidaire

(30) Priorität: 25.01.2005 AT 1082005; 02.09.2005 AT 14382005
(43) Veröffentlichungstag der Anmeldung: 26.07.2006
(73) Patentinhaber: AMI Agency for Medical Innovations GmbH, 6800 Feldkirch (AT)
(72) Erfinder: Scheyer, Mathias, Dr., 6800 Feldkirch (AT)
(74) Vertreter: Hofmann, Ralf U.

(56) Entgegenhaltungen:
- EP-A- 1 234 539
- DE-U1- 8 316 987
- US-A- 295 798
- US-A- 6 142 931
- US-A- 6 142 933

## Beschreibung

Die Erfindung betrifft eine Einrichtung zur Verwendung bei der Behandlung eines Hämorrhoidenprolaps durch Anbringung einer Raffungsnaht umfassend einen Tubus, der ein distales Ende, mit dem er voraus in ein Rektum eines Patienten einführbar ist, und ein offenes proximalles Ende und eine einen inneren Hohlraum umgebende Mantelwand aufweist, die mit einer länglichen Öffnung versehen ist, die sich in achsialer Richtung des Tubus erstreckt, und eine Verschlusseinrichtung, welche in einer Schließstellung die Öffnung schließt und von der die Öffnung sukzessive von distal nach proximal freigebbar ist und die ein Verschlussteil aufweist, innerhalb von dem der Tubus angeordnet ist und das von einem Teil eines Mantels eines Hohlzylinders gebildet wird und um die Längsachse des Tubus gegenüber dem Tubus verdrehbar ist.

Ein bekanntes Operationsverfahren besteht darin, im Bereich des ano-rektalen Übergangs Schnitte einzubringen und die freigelegten Hämorrhoiden-Arterien abzubinden und weiters vergrößerte Hämorrhoiden (prolabierende Noduli) zu entfernen. Diese Operation ist für den Patienten schmerzhaft, da sie im schmerzempfindlichen Bereich des ano-rektalen Übergangs stattfindet. Außerdem handelt es sich um eine Operation mit einer größeren Invasivität und den damit verbundenen Nachteilen und Risken.

Bekannt ist weiters die Durchführung einer minimal-invasiven Hämorrhoiden-Arterienligatur mittels einer in das Rektum einzuführenden Ultraschallsonde. Im distalen Bereich eines Tubus ist ein Ultraschallsensor angeordnet, und zwar neben einer Öffnung in der Mantelwand des Tubus. Mittels des Ultraschallsensors wird eine innerhalb der Darmwand verlaufende Hämorrhoiden-Arterie lokalisiert, wobei in der Folge eine Ligatur der Arterie durch die Öffnung im Tubus erfolgt. Ein Vorteil dieser heute häufig angewendeten Behandlungsmethode ist neben der minimalen Invasivität, dass die Behandlung im Bereich der Darmwand oberhalb der Linea Dentata erfolgt und somit nicht in einem schmerzempfindlichen Bereich. Allerdings kann diese Methode bei einem Hämorrhoidenprolaps des III und IV Grades häufig nicht mehr den gewünschten Behandlungserfolg bringen. Es handelt sich hierbei um ausgeprägte prolabierende Hämorrhoidalpolster (= prolabierende Noduli) bzw. prolabierende Mukosa, die mehr oder weniger ausgeprägt und mehr oder weniger permanent aus dem Anus prolabieren. Derartige Einrichtungen zur Hämorrhoiden-Arterienligatur sind beispielsweise aus der US 5,570,692 A und der EP 1 234 539 A2 bekannt.

Es wurden weiters vereinzelt bereits Operationen durchgeführt, bei welchen die prolabierenden Noduli mittels Raffungsnähten gerafft wurden, um den Prolaps wesentlich zu verringern. Diese Operationen haben sich jedoch als technisch schwierig erwiesen, da je nach Befund der Prolaps der Schleimhaut zielgerechte Durchstechungen in diesem Bereich verunmöglicht, die Einhaltung eines ausreichenden Abstands zur Linea Dentata nicht ausreichend kontrollierbar ist (Schmerzen sind die Folge) und der Zeitaufwand für die Einzeldurchstechungen zu groß ist (vier bis fünf Durchstechungen sind meist erforderlich, um zum gewünschten Erfolg zu gelangen). Diese Operationsmethode hat sich daher nicht durchgesetzt und gehört zu keiner der heute angewendeten Standardoperationsmethoden.

Eine Einrichtung mit den Merkmalen des Oberbegriffs des Patentanspruchs 1 ist aus der US 295 798 A bekannt. Diese Einrichtung bekannt. Diese Einrichtung dient insbesondere als Spekulum. Auch eine Behandlung der Darmwand ist mit dieser Einrichtung durchführbar.

Aufgabe der Erfindung ist es, eine Einrichtung bereitzustellen, die in vorteilhafter Weise zum einfachen Setzen einer Raffungsnaht eines Hämorrhoiden-Prolaps einsetzbar ist, wobei das Setzen der Raffungsnaht zumindest bis zu einem gewissen Grad, standardisiert wird. Erfindungsgemäß gelingt dies durch eine Einrichtung mit den Merkmalen des Patentanspruchs 1.

Zum Setzen einer Raffungsnaht wird der Tubus der Einrichtung in das Rektum eingeführt, wobei vorzugsweise ein Anschlag vorgesehen ist, der die Tiefe des Einführens begrenzt. Der Tubus wird hierbei derart um seine Längsachse gedreht, dass seine in Richtung der Längsachse des Tubus sich erstreckende längliche Öffnung im Bereich des Prolaps zu liegen kommt. Die Öffnung ist zunächst durch die Verschlusseinrichtung verschlossen.

Um die Naht auszuführen, wird ein erster distaler Abschnitt der Öffnung freigegeben, wobei die Schleimhaut des Prolaps sich in die Öffnung hineinwölbt. In diesem Bereich wird ein erster Stich der zu setzenden Raffungsnaht durchgeführt. In der Folge wird ein weiterer Abschnitt der Öffnung mittels der Verschlusseinrichtung freigegeben, der proximal an den zuvor freigegebenen Abschnitt der Öffnung anschließt. Es wird nunmehr in diesem proximal anschließenden Abschnitt eine weitere Durchstechung durchgeführt. Dieser Vorgang wird wiederholt, bis die gewünschte Anzahl von Durchstechungen durchgeführt worden ist. Jede der Durchstechungen kann sehr gut optisch überwacht werden und die Durchstechungen können in gewünschten Abständen vom Anus eingebracht werden. Die Gefahr, zu nahe zur Linea Dentata oder in Richtung zum Anus über diese hinaus zu gelangen, wird hierbei verringert. Auch sind die Durchstechungen einfach und rasch einbringbar. Das weitere Knüpfen der Naht kann in bekannter Weise erfolgen.

Vorteilhafterweise ist eine Beleuchtungseinrichtung zur Beleuchtung des Operationsbereiches vorhanden.

Die Verschlusseinrichtung weist erfindungsgemäß ein den Tubus außen umgebendes Verschlussteil auf, das von einem Teil eines Mantels eines Hohlzylinders gebildet wird und das um die Längsachse dieses Hohlzylinders bzw. des Tubus gegenüber dem Tubus verdrehbar ist. Je nach Drehstellung des Verschlussteils wird ein mehr oder weniger großer distaler Abschnitt der länglichen Öffnung in der Mantelwand des Tubus freigegeben oder die Öffnung wird verschlossen.

Am Tubus ist ein Ultraschallsensor angeordnet und der Tubus ist mit einer Ligaturöffnung versehen. Die Einrichtung kann somit auch dazu eingesetzt werden, um intramurale Arterien zu ligieren. Eine solche Ligatur geht dem Setzen einer Raffungsnaht üblicherweise voraus. Vorteilhafterweise ist die Ligaturöffnung ebenfalls von der Verschlusseinrichtung verschließbar und freigebbar. Die Ligaturöffnung kann als separate Öffnung im Tubus oder als seitliche Erweiterung der achsialen Öffnung ausgebildet sein.

Die Begriffe distal und proximal in Bezug auf die erfindungsgemäße Einrichtung sind so zu verstehen, dass diese auf die Seite bezogen sind, die dem Operateur zugewandt ist, d. h. das distale Ende der Einrichtung ist das Einführende des Tubus und das proximale Ende ist das gegenüberliegende Ende der Einrichtung. Auf den Gastro-Intestinal-Trakt bezogen bezeichnen die Begriffe proximal und distal dagegen die Lage relativ zum Mund des Patienten. Der Tubus wird somit in das distale Rektum eingeführt.

Als Hämorrhoidenprolaps wird im Rahmen dieser Schrift jeder Prolaps der Schleimhaut des distalen Rektums bzw. des "Cavernosum recti" bezeichnet. Solche prolabierende Schleimhaut wird in dieser Schrift auch als "Mukosaprolaps", "prolabierender Knoten", "prolabierender Nodulus", "prolabierendes Hämorrhoidalpolster" oder "prolabierender Fortsatz" bezeichnet.

Weitere Vorteile und Einzelheiten der Erfindung werden im Folgenden anhand der beiliegenden Zeichnung erläutert. In dieser zeigen:
- Fig. 1 und Fig. 2: Schrägsichten von schräg hinten und schräg vorne einer Ausführungsform der Erfindung, in der Ligaturstellung der Verschlusseinrichtung (Fig. 1 zeigt die Einrichtung ohne den Handgriff und in Fig. 2 ist nur ein Abschnitt des Handgriffs dargestellt);
- Fig. 3: und Fig. 4 Schrägsichten aus verschiedenen Blickrichtungen im auseinandergezogenen Zustand des Tubus und der Verschlusseinrichtung;
- Fig. 5: die Einrichtung in der Schließstellung;
- Fig. 6: eine Zwischenstellung und
- Fig. 7: die Einrichtung in der Offenstellung der Verschlusseinrichtung, in der die achsiale Öffnung im Tubus vollständig freigegeben ist.

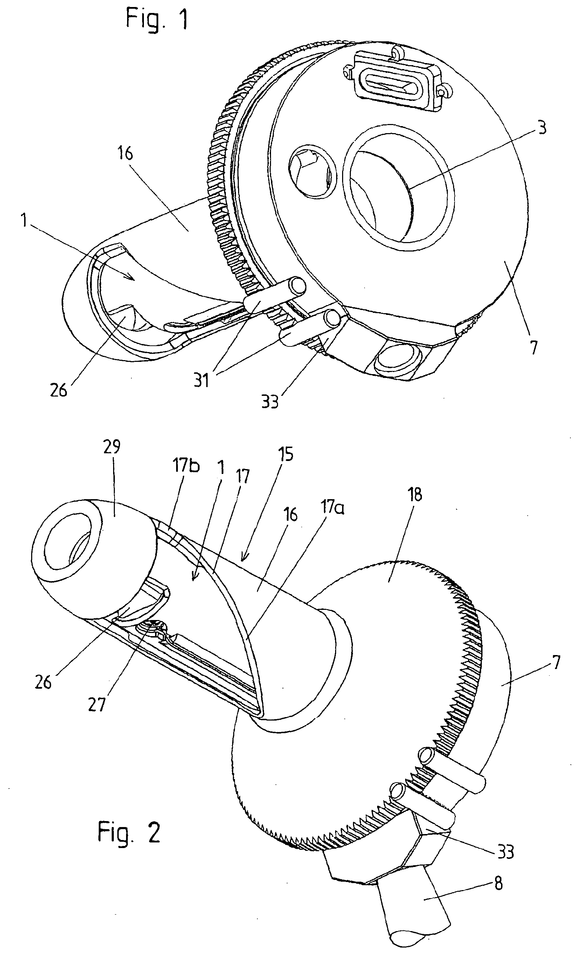

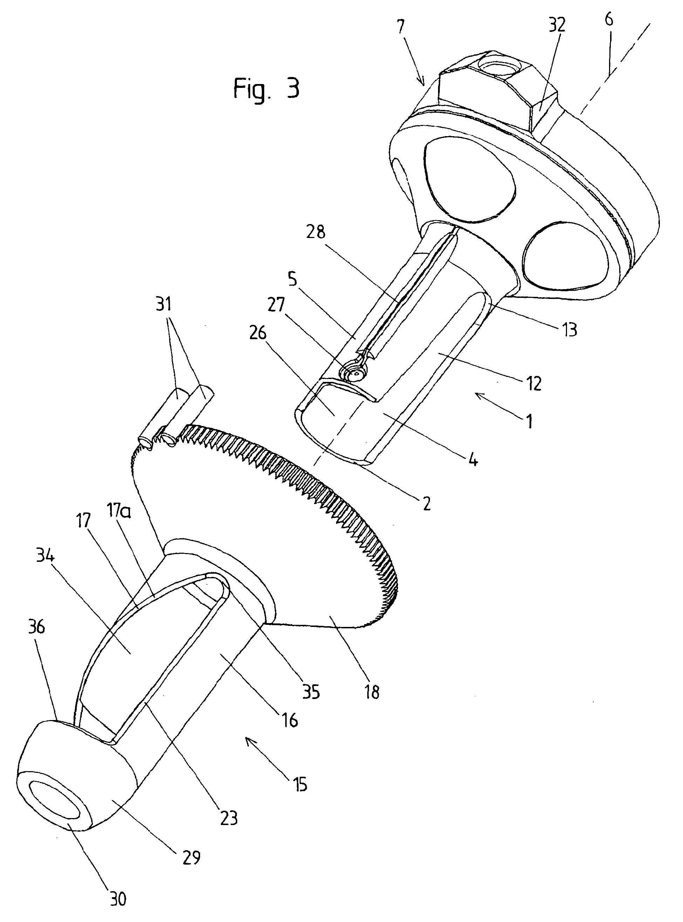

Ein Ausführungsbeispiel einer erfindungsgemäßen Einrichtung ist in den Fig. 1 bis 7 dargestellt. Die Einrichtung umfasst einen Tubus 1 (= eine Hülse) und eine weiter unten genauer erläuterte, um die Längsachse 6 des Tubus 1 drehbar angeordnete Verschlusseinrichtung 15.

Der Tubus 1 besitzt eine den inneren Hohlraum 4 des Tubus 1 umgebende Mantelwand 5. Diese ist im Querschnitt gesehen in Form eines Kreisrings, bzw. im Bereich, in welchem die weiter unten beschriebene Öffnung 12 angeordnet ist, in Form eines Umfangsabschnitts eines Kreisrings ausgebildet und umgibt hierbei die Längsachse 6 des Tubus 1.

Das proximale Ende 3 des Tubus 1 ist offen ausgebildet und in diesem Ausführungsbeispiel ist der Tubus 1 auch an seinem distalen Ende 2 offen ausgebildet. Der Tubus 1 ist an einer Halterung 7 angebracht, die einen Handgriff 8 aufweist (nur in Fig. 2 teilweise dargestellt). Die Halterung 7 besitzt einen inneren Hohlraum, der den inneren Hohlraum 4 des Tubus 1 fortsetzt.

Unterschiedliche Befestigungen des Tubus 1 an der Halterung 7 sind denkbar und möglich. Ebenso sind einstückige Ausbildungen des Tubus 1 mit der Halterung 7 denkbar und möglich. Die Einrichtung kann sterilisierbar oder als Einmalinstrument ausgebildet sein.

Der Tubus 1 besitzt einen (zumindest im Wesentlichen) zylindrischen Abschnitt 24 und im Bereich seines proximalen Endes 3 einen sich in seinem Umfang erweiternden Abschnitt 25 (der zylindrische Abschnitt 24 kann sich auch innerhalb des Abschnitts 25 fortsetzen).

Die Mantelwand 5 des Tubus 1 besitzt eine in achsialer Richtung des Tubus 1 sich erstreckende längliche Öffnung 12, die zum distalen Ende 2 des Tubus 1 hin offen ist. Das distale Ende der Öffnung 12 fällt also mit dem distalen Ende 2 des Tubus 1 zusammen. Das proximale Ende 13 der Öffnung 12 befindet sich in einem proximalen Bereich des zylindrischen Abschnitts 24.

Diese längliche Öffnung 12 könnte auch als Langloch oder Schlitz bezeichnet werden. Die Öffnung 12 verläuft vorzugsweise über den Großteil der Länge des Tubus 1.

Vorzugsweise ist in achsialer Richtung des Tubus 1 gemessene Länge der Öffnung 12 mindestens doppelt so groß wie ihre senkrecht hierzu gemessene Breite. Günstigerweise liegt die Länge der Öffnung 12 im Bereich von 40 bis 80 mm. Ihre Breite liegt günstigerweise im Bereich von 8 bis 20 mm.

Die Breite der Öffnung 12 kann sich über ihre achsiale Ausdehnung ändern, vorzugsweise in proximaler Richtung etwas verringern.

Die über die Halterung 7 vorstehende achsiale Länge des Tubus 1 liegt vorzugsweise im Bereich von 60 bis 100 mm.

In einem an das distale Ende 2 des Tubus 1 anschließenden Bereich besitzt die achsiale Öffnung 12 eine seitliche Erweiterung, die eine Ligaturöffnung 26 bildet. Diese Ligaturöffnung 26 ist ebenfalls zum distalen Ende 2 des Tubus 1 hin offen.

Denkbar und möglich wäre es auch, dass die achsiale Öffnung 12 und/oder die Ligaturöffnung 26 nicht bis zum distalen Ende 2 des Tubus 1 verlaufen, also als Fensteröffnungen ausgebildet sind. Die Öffnung 12 und die Ligaturöffnung 26 können auch voneinander separiert sein.

An der Mantelwand 5 des Tubus 1 ist ein Ultraschallsensor 27 angebracht und wird über eine entlang der Mantelwand 5 verlaufende elektrische Leitung 28 angesteuert. Der Ultraschallsensor 27 ist proximal neben der Ligaturöffnung 26 angeordnet.

Die insgesamt hülsenförmig ausgebildete Verschlusseinrichtung 15 umfasst ein Verschlussteil 16, an welches proximal ein sich erweiternder, im Querschnitt ringförmiger Abschnitt 18 anschließt. Distal schließt an das Verschlussteil 16 ein distaler ringförmiger Abschnitt 29 an, der das distale Ende 30 der Verschlusseinrichtung bildet, wobei er sich zum distalen Ende 30 der Verschlusseinrichtung 15 hin etwas verjüngt.

Am Abschnitt 18 der Verschlusseinrichtung 15 sind Anschlagelemente 31 angeordnet, die mit Anschlägen 32, 33 der Halterung 7 zusammenwirken.

Das Verschlussteil 16 ist in Form eines Teils des Mantels eines Hohlzylinders ausgebildet. Anders ausgedrückt ist das Verschlussteil 16 in Form eines Hohlzylinders mit einer Öffnung 34 in seinem Mantel ausgebildet. Ein Rand 17 dieser Öffnung 34 weist einen schraubenartig um die Längsachse des Verschlussteils 16 verlaufenden Abschnitt 17a und einen distal daran anschließenden achsial verlaufenden Abschnitt 17b auf. Der Rand 23 der Öffnung 34 verläuft in achsialer Richtung. Der Abschnitt 17a des Randes 17 ist proximal mit dem Rand 23 über einen gebogenen Rand 35 der Öffnung 34 verbunden. Der Abschnitt 17b des Randes 17 und der Rand 23 verlaufen bis zum distalen Ende des Verschlussteils 16 und sind dort über den proximalen Rand 36 des Abschnitts 29 der Verschlusseinrichtung miteinander verbunden.

Die Öffnung 34 stellt somit im gezeigten Ausführungsbeispiel eine Fensteröffnung im Mantel der insgesamt hülsenförmigen Verschlusseinrichtung 15 dar.

Die Umfangserstreckung des Verschlussteils 16 (also des Mantels des Hohlzylinders, der das Verschlussteil 16 bildet) ist über den achsialen Bereich, in dem sich die achsiale Öffnung 12 im Tubus 1 erstreckt, zumindest so groß wie die Umfangserstreckung der Öffnung 12 und diese Umfangserstreckung des Verschlussteils 16 nimmt zum proximalen Ende des Verschlussteils 16 hin zu.

Die Verschlusseinrichtung 15 ist in diesem Ausführungsbeispiel auf den Tubus 1 von seinem distalen Ende 2 her aufsteckbar, wobei im Benutzungszustand der Einrichtung das Verschlussteil 16 den Tubus 1 außen über einen mehr oder weniger großen Teil seines Umfangs umgibt. Der Innendurchmesser des Verschlussteils 16 entspricht hierbei zuzüglich eines Spiels zur Ausbildung einer Gleitführung auf dem Tubus 1 dem Außendurchmesser des vom Verschlussteil 16 umgebenen Abschnitts des Tubus 1. Das Spiel beträgt günstigerweise weniger als 1 mm, wobei ein Wert von weniger als 0,3 mm bevorzugt ist. Im Benutzungszustand der Einrichtung liegen der Tubus 1 und das Verschlussteil 16 koachsial zueinander.

Die Verschlusseinrichtung 15 mit dem Verschlussteil 16 ist gegenüber dem Tubus 1 um seine Längsachse bzw. die Längsachse des Tubus 1 verdrehbar. Zur Erleichterung der Verdrehung besitzt der erweiterte Abschnitt 18 proximal einen geriffelten Rand. Je nach Drehstellung werden hierbei die folgenden Stellungen eingenommen:

In der Drehstellung, in der ein Anschlagelement 31 der Verschlusseinrichtung 15 am Anschlag 33 der Halterung 7 anliegt, ist die achsiale Öffnung 12 im Tubus 1 vom Verschlussteil 16 verschlossen, aber die Ligaturöffnung 26 im Tubus 1 ist freigegeben. Diese Stellung wird als "Ligaturstellung" bezeichnet (vgl. Fig. 1 und 2).

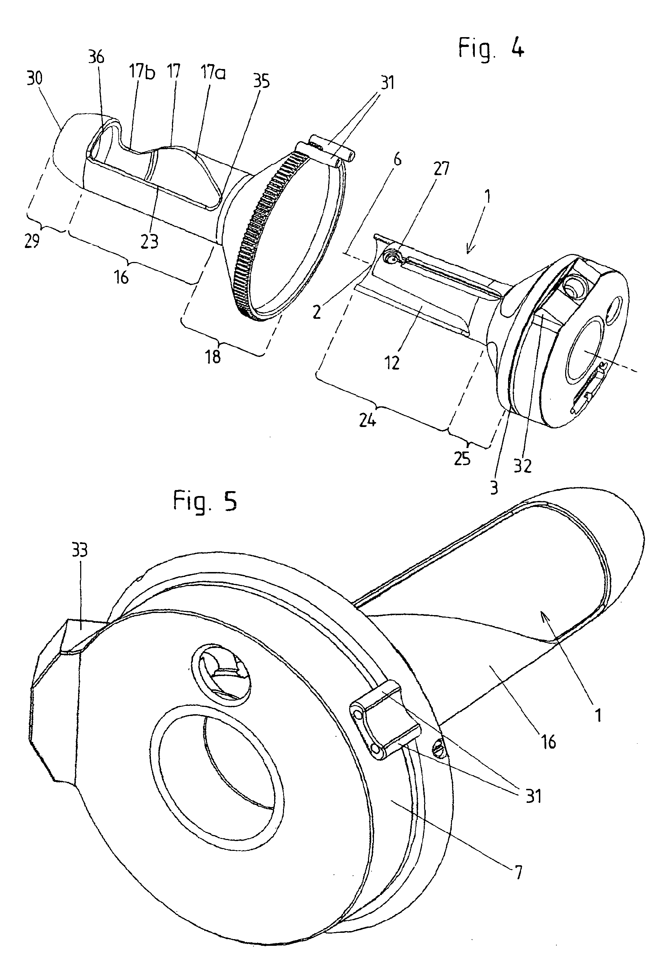

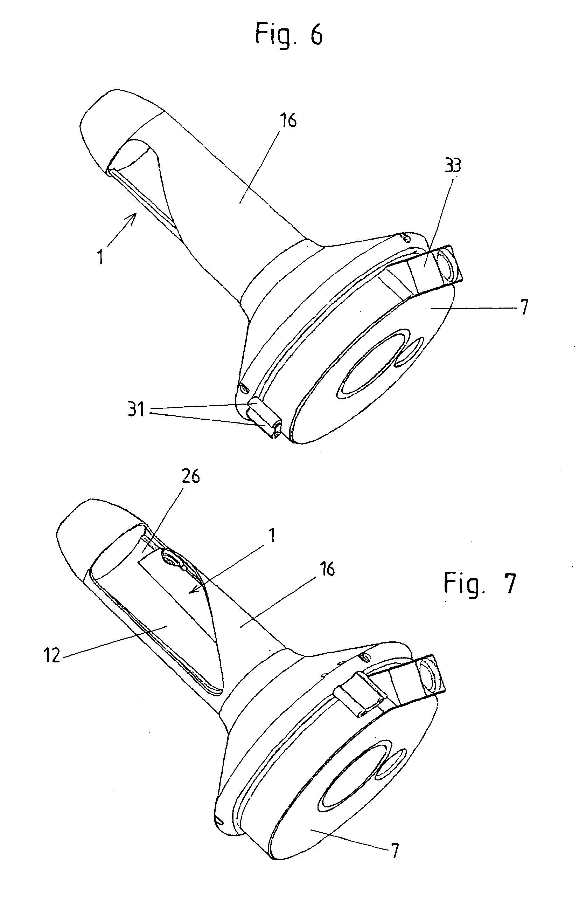

Im vollständig in die andere Endstellung verdrehten Zustand der Verschlusseinrichtung 15, in welchem ein Anschlagelement 31 am Anschlag 32 der Halterung 7 anliegt, sind sowohl die achsiale Öffnung 12 als auch die Ligaturöffnung 26 des Tubus 1 vom Verschlussteil 16 abgedeckt und diese Stellung wird als "Schließstellung" bezeichnet. Diese Stellung ist in Fig. 5 dargestellt. In Fig. 5 ist der Abschnitt 18 der Verschlusseinrichtung hierbei geringfügig modifiziert dargestellt, insbesondere sind die beiden Anschlagelemente 31 als ein Stück ausgebildet und die Riffelung am Rand ist nicht dargestellt. Dies trifft auch auf Fig. 6 und Fig. 7 zu.

Wenn die Verschlusseinrichtung 15 ausgehend von der Schließstellung in Richtung zur Ligaturstellung gedreht wird, so wird die achsiale Öffnung 12 des Tubus 1 zunehmend von distal nach proximal freigegeben, bis sie über ihre ganze Länge freigegeben ist und die "Offenstellung" erreicht ist (vgl. Fig. 7). Die Stellungen, in denen nur ein distaler Abschnitt der achsialen Öffnung 12 des Tubus 1 freigegeben ist, werden als "Zwischenstellungen" bezeichnet. Eine Zwischenstellung ist in Fig. 6 dargestellt.

Die Einrichtung weist vorteilhafterweise eine Beleuchtungseinrichtung auf (nicht dargestellt in den Fig. 1 bis 7). Beispielsweise sind in der Halterung 7 mehrere in Umfangsrichtung des Tubus 1 voneinander beabstandete Leuchtdioden 21 angeordnet, die in das proximale Ende des erweiterten Abschnitts 25 des Tubus 1 einstrahlen und deren Licht in der Mantelwand 5 des Tubus 1 durch Totalreflexion weitergeleitet wird. Gewünschte Austrittsstellen für das Licht können z. B. durch Aufrauungen der Innenfläche der Mantelwand ausgebildet werden, um den Operationsbereich im Bereich der Öffnung 12 zu beleuchten. Auch andere Beleuchtungseinrichtungen sind denkbar und möglich, wobei das Licht beispielsweise mittels eines oder mehrerer Lichtleiter im Tubus 1 bis in die Nähe der Operationsstelle geführt sein kann. Es kann auch eine externe Lichtquelle vorgesehen sein, von der Licht durch ein Lichtleitkabel in die Einrichtung eingeleitet wird.

Mit der in den Fig. 1 bis 7 dargestellten Einrichtung kann nicht nur eine Raffungsnaht durchgeführt werden, sondern die Einrichtung kann auch für eine vorausgehende Ligatur von Hämorrhoidalarterien bzw. intramuralen Arterien eingesetzt werden.

Hierbei wird der Tubus 1 mit den diesen außen umgebenden Teilen der Verschlusseinrichtung 15 in das Rektum eingeführt, und zwar in einer Drehstellung entsprechend der Lage eines zu behandelnden Schleimhautvorfalls. Die Verschlusseinrichtung befindet sich hierbei in ihrer Schließstellung. Der erweiterte Abschnitt 18 der Verschlusseinrichtung 15 bildet im gezeigten Ausführungsbeispiel einen Anschlag zur Begrenzung des Einführens des Tubus 1.

Zur anschließenden Ligatur von intramuralen (=in der Darmwand liegende) Arterien wird die Verschlusseinrichtung in die Ligaturstellung gedreht. Die Ligatur wird in herkömmlicher Weise durch Detektion von entsprechenden Arterien mittels des Ultraschallsensors 27 (und einer entsprechenden Ansteuer- und Auswertelektronik) durch die Ligaturöffnung 26 durchgeführt, um die Blutzufuhr zum "Cavernosum recti" zu reduzieren.

In der Folge wird durch Verdrehen der Verschlusseinrichtung die achsiale Öffnung 12 schrittweise von ihrem distalen Ende 14 her freigegeben, wobei vom Chirurgen eine fortlaufende Naht gelegt wird. Diese ergreift die Mukosa, die Submukosa und evtl. auch die "Muscularis mukosae". Mittels schlankem Nadelhalter und entsprechend gekrümmter Nadel wird durch den inneren Hohlraum 4 des Tubus 1 gearbeitet. Die fortlaufende Naht wird schrittweise über eine Länge von 4 bis 6 cm angelegt, bis in die Nähe der Linea dentata. Durch Knoten der fortlaufenden Naht in das distale Rektum erfolgt ein Repositionieren (bzw. ein Lifting bzw. eine Raffung) und ein Fixieren des Mukosaprolapses und eine anantomische Rekonstruktion des Corpus covernosum recti.

Nach dem Verknoten der fortlaufenden Naht im Bereich des distalen Endes 14 der achsialen Öffnung 12 wird die Einrichtung aus dem Rektum entfernt, die Verschlusseinrichtung 15 in die Schließstellung gebracht und die Einrichtung neuerlich eingeführt, um den nächsten prolabierenden Schleimhautvorfall in derselben Weise zu behandeln. Dies wird wiederholt, bis über den Umfang des Rektums alle Schleimhautvorfälle behandelt worden sind.

Während der Operation ist es für den Chirurgen auch möglich, ein Instrument durch das offene distale Ende der Einrichtung durchzuführen und mit diesem Instrument prolabierte Schleimhaut in die distale Richtung zu drücken, um die Raffung zu unterstützen und die Naht zu entlasten.

Eine am distalen Ende geschlossene Ausbildung des Tubus 1 und/oder der Verschlusseinrichtung 15 ist denkbar und möglich, aber weniger bevorzugt.

Zur definierten Einstellung der Drehstellungen der Verschlusseinrichtung 15 kann eine zwischen der Halterung 7 und der Verschlusseinrichtung 15 wirkende Rasteinrichtung vorhanden sein.

Unterschiedliche weitere Modifikationen der beschriebenen Ausführungsbeispiele sind denkbar und möglich, ohne den Bereich der Erfindung, wie er in den Ansprüchen definiert ist, zu verlassen.

### Legende

### zu den Hinweisziffern:

- 1: Tubus
- 2: distales Ende des Tubus
- 3: proximales Ende des Tubus
- 4: innerer Hohlraum des Tubus
- 5: Mantelwand des Tubus
- 6: Längsachse
- 7: Halterung
- 8: Handgriff
- 12: Öffnung des Tubus
- 13: proximales Ende der Öffnung
- 15: Verschlusseinrichtung
- 16: Verschlussteil
- 17: Rand
- 17a: Abschnitt des Randes
- 17b: Abschnitt des Randes
- 18: Abschnitt der Verschlusseinrichtung
- 23: Rand
- 24: zylindrischer Abschnitt des Tubus
- 25: Abschnitt des Tubus
- 26: Ligaturöffnung
- 27: Ultraschallsensor
- 28: elektrische Leitung
- 29: distaler Abschnitt
- 30: distales Ende der Verschlusseinrichtung
- 31: Anschlagelement
- 32: Anschlag
- 33: Anschlag
- 34: Öffnung im Verschlussteil
- 35: Rand
- 36: Rand

## Patentansprüche

1. Einrichtung zur Verwendung bei der Behandlung eines Hämorrhoidenprolaps durch Anbringung einer Raffungsnaht umfassend einen Tubus (1), der ein distales Ende (2), mit dem er voraus in ein Rektum eines Patienten einführbar ist, und ein offenes proximales Ende (3) und eine einen inneren Hohlraum (4) umgebende Mantelwand (5) aufweist, die mit einer länglichen Öffnung (12) versehen ist, die sich in achsialer Richtung des Tubus (1) erstreckt, und eine Verschlusseinrichtung (15), welche in einer Schließstellung die Öffnung (12) schließt und von der die Öffnung (12) sukzessive von distal nach proximal freigebbar ist und die ein Verschlussteil (16) aufweist, innerhalb von dem der Tubus (1) angeordnet ist und das von einem Teil eines Mantels eines Hohlzylinders gebildet wird und um die Längsachse (6) des Tubus (1) gegenüber dem Tubus (1) verdrehbar ist, **dadurch gekennzeichnet, dass** am Tubus (1) ein Ultraschallsensor (27) angeordnet ist und der Tubus (1) eine Ligaturöffnung (26) aufweist, wobei der Ultraschallsensor (27) proximal neben der Ligaturöffnung (26) angeordnet ist.

2. Einrichtung nach Anspruch 1, **dadurch gekennzeichnet, dass** die längliche Öffnung (12) des Tubus (1) von der Verschlusseinrichtung (15) kontinuierlich freigebbar ist.

3. Einrichtung nach Anspruch 1 oder Anspruch 2, **dadurch gekennzeichnet, dass** die Mantelwand (5) des Tubus (1) zumindest über einen Abschnitt (24) des Tubus (1), zylindrisch ausgebildet ist.

4. Einrichtung nach Anspruch 3, **dadurch gekennzeichnet, dass** der zylindrische Abschnitt (24) des Tubus (1) den Großteil der Längserstreckung des Tubus (1) ausmacht.

5. Einrichtung nach einem Ansprüche 1 bis 4, **dadurch gekennzeichnet, dass** der Innendurchmesser des Verschlussteils (16) zuzüglich einem Spiel zur Ausbildung einer Gleitführung auf dem Tubus (1) dem Außendurchmesser des vom Verschlussteil (16) umgebenen Abschnitts des Tubus (1) entspricht.

6. Einrichtung nach einem der Ansprüche 1 bis 5, **dadurch gekennzeichnet, dass** die Umfangserstreckung des Verschlussteils (16) im achsialen Bereich der Öffnung (12) des Tubus (1) zumindest der Breite (b) der Öffnung (12) entspricht und die Öffnung (12) des Tubus (1) in einer Drehstellung des Verschlussteils (16) bezüglich des Tubus (1) vom Verschlussteil (16) vollständig abgedeckt ist und durch eine Verdrehung des Verschlussteils (16) gegenüber dem Tubus (1) kontinuierlich von distal nach proximal freigebbar ist.

7. Einrichtung nach einem der Ansprüche 1 bis 6, **dadurch gekennzeichnet, dass** das Verschlussteil (16) einen schraubenförmig um die Längsachse (6) des Tubus (1) verlaufenden Rand (17; 17a) aufweist.

8. Einrichtung nach Anspruch 7, **dadurch gekennzeichnet, dass** das Verschlussteil (16) einen in achsialer Richtung sich erstreckenden Rand (23) aufweist.

9. Einrichtung nach einem der Ansprüche 1 bis 8, **dadurch gekennzeichnet, dass** der Tubus (1) an einer Halterung (7) angebracht ist, welche einen Handgriff (8) aufweist.

10. Einrichtung nach einem der Ansprüche 1 bis 9, **dadurch gekennzeichnet, dass** eine Beleuchtungseinrichtung zur Beleuchtung des Operationsbereichs vorhanden ist.

11. Einrichtung nach einem der Ansprüche 1 bis 10, **dadurch gekennzeichnet, dass** ein Anschlag zur Begrenzung des Einführens des Tubus (1) in das Rektum vorhanden ist.

12. Einrichtung nach einem der Ansprüche 1 bis 11, **dadurch gekennzeichnet, dass** die Ligaturöffnung (26) von der Verschlusseinrichtung (15) verschließbar und freigebbar ist.

13. Einrichtung nach einem der Ansprüche 1 bis 12, **dadurch gekennzeichnet, dass** die Ligaturöffnung (26) in Form einer seitlichen Erweiterung der achsialen Öffnung (12) im Tubus (1) im Bereich des distalen Endes (14) der achsialen Öffnung (12) ausgebildet ist.

## Claims

1. A device for use during the treatment of a haemorrhoid prolapse by application of a tightening seam comprising a tube (1), which has a distal end (2), by which it is capable of being inserted in advance into the rectum of a patient, and an open proximal end (3) and a casing wall (5), which surrounds an internal cavity (4) and which is provided with an elongate opening (12) which extends in the axial direction of the tube (1), and a closure device (15) which closes the opening (12) in a closure position and by which the opening (12) is capable of being exposed in succession from distal to proximal and which has a closure part (16), inside which the tube (1) is arranged and which is formed by part of a casing of a hollow cylinder and is rotatable about the longitudinal axis (6) of the tube (1) with respect to the tube (1), **characterized in that** an ultrasonic sensor (27) is arranged on the tube (1), and the tube (1) has a ligature opening (26), wherein the ultrasonic sensor (27) is arranged proximally adjacent to the ligature opening (26).

2. A device according to Claim 1, **characterized in that** the elongate opening (12) of the tube (1) is capable of being exposed in a continuous manner by the closure device (15).

3. A device according to Claim 1 or Claim 2, **characterized in that** the casing wall (5) of the tube (1) is made cylindrical, at least over a portion (24) of the tube (1).

4. A device according to Claim 3, **characterized in that** the cylindrical portion (24) of the tube (1) makes up the greater part of the longitudinal extension of the tube (1).

5. A device according to any one [of] Claims 1 to 4, **characterized in that** the internal diameter of the closure part (16) plus a degree of clearance in order to form a sliding guide on the tube (1) corresponds to the external diameter of the portion of the tube (1) surrounded by the closure part (16).

6. A device according to any one of Claims 1 to 5, **characterized in that** the peripheral extension of the closure part (16) in the axial region of the opening (12) of the tube (1) corresponds at least to the width (b) of the opening (12), and the opening (12) of the tube (1) is completely covered with respect to the tube (1) by the closure part (16) in a rotational position of the closure part (16), and is capable of being exposed in a continuous manner from distal to proximal by a rotation of the closure part (16) with respect to the tube (1).

7. A device according to any one of Claims 1 to 6, **characterized in that** the closure part (16) has an edge (17; 17a) extending in a helical manner about the longitudinal axis (6) of the tube (1).

8. A device according to Claim 7, **characterized in that** the closure part (16) has an edge (23) extending in the axial direction.

9. A device according to any one of Claims 1 to 8, **characterized in that** the tube (1) is attached to a holding means (7) which has a handle (8).

10. A device according to any one of Claims 1 to 9, **characterized in that** an illumination device is provided in order to illuminate the operation area.

11. A device according to any one of Claims 1 to 10, **characterized in that** a stop is provided in order to limit the insertion of the tube (1) into the rectum.

12. A device according to any one of Claims 1 to 11, **characterized in that** the ligature opening (26) is capable of being closed and exposed by the closure device (15).

13. A device according to any one of Claims 1 to 12, **characterized in that** the ligature opening (26) is constructed in the form of a lateral extension of the axial opening (12) in the tube (1) in the region of the distal end (14) of the axial opening (12).

## Revendications

1. Dispositif destiné à être utilisé pour traiter un prolapsus hémorroïdaire en pratiquant une suture de compression, constitué d'un tube (1) qui comporte une extrémité distale (2) par laquelle il est introduit préalablement dans le rectum d'un patient et une extrémité proximale ouverte (3), d'une enveloppe (5) entourant un espace creux intérieur (4), munie d'une ouverture allongée (12) s'étendant dans la direction axiale du tube (1), ainsi que d'un dispositif de fermeture (15) qui, à une position de fermeture, ferme l'ouverture (12) et dont l'ouverture (12) peut être dégagée progressivement de la partie distale à la partie proximale, et qui présente une partie de fermeture (16) logeant le tube (1) et, constituée par une partie d'enveloppe d'un cylindre creux et pouvant tourner autour de l'axe longitudinal (6) du tube (1) par rapport au tube (1),
**caractérisé en ce qu'**
un capteur ultrasonore (27) est disposé sur le tube (1) et le tube présente une ouverture pour ligature (26), le capteur ultrasonore (27) étant à un emplacement proximal près de l'ouverture pour ligature (26).

2. Dispositif selon la revendication 1,
**caractérisé en ce que**
l'ouverture allongée (12) du tube (1) peut être dégagée en continu du dispositif de fermeture (15).

3. Dispositif selon la revendication 1, ou la revendication 2,
**caractérisé en ce que**
l'enveloppe (5) du tube (1) est, tout au moins sur un segment (24) du tube (1), de forme cylindrique.

4. Dispositif selon la revendication 3,
**caractérisé en ce que**
le segment cylindrique (24) du tube (1) forme la majeure partie de l'étendue longitudinale du tube (1).

5. Dispositif selon l'une des revendications 1 à 4,
**caractérisé en ce que**
le diamètre intérieur de la partie de fermeture (16), avec en plus un jeu pour former un guidage coulissant sur le tube (1), est égal au diamètre extérieur du segment du tube (1) entouré par la partie de fermeture (16).

6. Dispositif selon l'une des revendications 1 à 5,
**caractérisé en ce que**
l'étendue périphérique de la partie de fermeture (16) est égale, dans la zone axiale de l'ouverture (12) du tube (1), à au moins la largeur (b) de l'ouverture (12) et l'ouverture (12) du tube (1), dans une position de rotation de la partie de fermeture (16) par rapport au tube (1), est totalement recouverte par la partie de fermeture (16) et, par une rotation de la partie de fermeture (16) par rapport au tube (1), peut être dégagée en continu de la partie distale à la partie proximale.

7. Dispositif selon l'une des revendications 1 à 6,
**caractérisé en ce que**
la partie de fermeture (16) présente un bord (17, 17a) s'étendant en forme hélicoïdale autour de l'axe longitudinal (6) du tube (1).

8. Dispositif selon la revendication 7,
**caractérisé en ce que**
la partie de fermeture (16) présente un bord (23) s'étendant dans la direction axiale.

9. Dispositif selon l'une des revendications 1 à 8,
**caractérisé en ce que**
le tube (1) est monté sur un support (7) qui possède une poignée (8).

10. Dispositif selon l'une des revendications 1 à 9,
**caractérisé en ce qu'**
il existe un système d'éclairage pour éclairer la région de l'opération.

11. Dispositif selon l'une des revendications 1 à 10,
**caractérisé par**
une butée pour limiter l'introduction du tube (1) dans le rectum.

12. Dispositif selon l'une des revendications 1 à 11,
**caractérisé en ce que**
l'ouverture pour ligature (26) peut être fermée par le dispositif de fermeture (15) et dégagée de celui-ci.

13. Dispositif selon l'une des revendications 1 à 12,
**caractérisé en ce que**
l'ouverture pour ligature (26) est en forme d'élargissement latéral de l'ouverture axiale (12) dans le tube (1) dans la région de l'extrémité distale (14) de l'ouverture axiale (12).
